# EUROPEAN PATENT APPLICATION

(11) **EP 1 808 198 A1**
(43) Date of publication of application: **18.07.2007**
(21) Application number: 05805390.1
(22) Date of filing: 31.10.2005
(51) Int. Cl.: A61N 5/06

(54) **PHOTODYNAMIC THERAPY APPARATUS**

(30) Priority: 02.11.2004 JP 2004319343
(71) Applicant: Keio University, Tokyo 108-8345 (JP); TERUMO KABUSHIKI KAISHA, Tokyo 151-0072 (JP)
(72) Inventor: Arai, Tsunenori Keio Univ. Fac. of Science & Tech., Yokohama-shi, Kanagawa ; 2230061 (JP); Ohmori, Sayaka Keio Univ. Fac. of Sciece & Techn., Yokohama-shi, Kanagawa; 2230061 (JP); Shiono, Hiroshi c/o Terumo Kabushiki Kaisha, Kanagawa; 2590151 (JP)
(74) Representative: Casalonga, Axel
(86) International application number: PCT/JP2005/020015
(87) International publication number: WO 2006/049132

(57) **Abstract**

Provided is the photodynamic therapy apparatus which is capable of detecting damage to the healthy part existing at a shallower part of a living body than the lesioned part so as to prevent disorder of the healthy part. The photodynamic therapy apparatus 10 is the photodynamic therapy apparatus 10 for treating the lesioned part existing at a deep part of a living body, using a light-sensitive substance which is activated by a light having a peak intensity within a predetermined range, but almost not activated by a light having the peak intensity out of the predetermined range, having: an irradiation device 12 which irradiates', onto the living body, pulsed light having wavelength which is capable of activating the light-sensitive substance; a detection device 14 which detects activation of the light-sensitive substance; and a control means 16 which controls the peak intensity of the light, based on result of detection obtained by the detection device 14 so that the light-sensitive substance is not activated at the healthy part existing at a shallower part than the lesioned part, and the light which reaches the lesioned part has the peak intensity within the predetermined range.

## Description

### Technical Field

The present invention relates to a photodynamic therapy apparatus, in particular, relates to the photodynamic therapy apparatus which damages only a deep lesioned part while preserving a healthy part existing in a shallower part than the lesioned part, without damaging, in treatment of the deep lesioned part of a living body.

### Background Art

Applications of a photodynamic therapy (PDT) to various treatments in addition to the endoscopic treatment of early stage cancer are studied. A PDT is a treatment method, in which a photosensitive substance (photo-sensitizer) such as a porphyrin derivative or the like is administered by a method of intravenous injection or the like, and subjected to be selectively absorbed and accumulated at a lesioned tissue part like a cancer tissue, as a treatment target, and subsequently a light beam such as a laser beam is irradiated to damage the lesioned tissue part.

PDT utilizes a selective accumulation property to a lesioned part, and a light-induced sensitization property of a photosensitizer. Mechanismof PDT is advocatedas follows. The photosensitizer captured in the lesioned part is excited by irradiation of light beams. Energies of the photosensitizer thus exited are transferred to oxygen existing in the lesioned part to generate activated singlet oxygen (free radical). And the resultant singlet oxygen necrotizes cells in the lesioned part by strong oxidative ability thereof. PDT treatment by such free radical is called a type II reaction.

In such a PDT treatment, there is a method for detecting generation of free radical during treatment, and when generation of predetermined amount of free radical is detected, it is determined that the treatment is completed, and treatment is terminated (for example, see Patent Literature 1: US 6,128,525).

However, by only detection of generation amount of free radical simply, it is determined that the treatment is completed, based on amount of free radical generated, even in the case of treatment of any part of the living body; resulting in, for example, determination of the termination of treatment without sufficient treatment at the lesioned part, based on detection of predetermined amount of free radical, even in the case where a photosensitizer has reacted at the healthy part at a shallow part of the living body instead of the lesioned part, when the lesioned part is existing at deep part of the living body. As a result, there is a possibility that treatment could end up causing the healthy part to suffer from undesirable damages. Moreover, it is impossible to detect such damages of healthy part that needs no treatment.

In addition, in the case where the lesioned part is existing at deep part of the living body, a method is known for treatment only the lesioned part while preserving the healthy part at a shallow part in the living body (for example, see Patent Literature 2). This method provides activation of a photosensitizer only at the lesioned part, by focusing a laser beam at the lesioned part.

However, even in this method, the healthy part at a shallow part in the living body could be damaged, in case a laser beam is out of focus, or laser intensity or concentration of the medical agent is varied; and detection of damage at the healthy part is impossible.
Patent Literature 1: USP No. 6,128,525
Patent Literature 2: USP No. 5,829,448

### Problems to be solved by the Invention

The present invention has been proposed under the above situation. It is an object of the present invention to provide the photodynamic therapy apparatus, which is capable of detecting damage to the healthy part existing at shallower part of a living body than the lesioned part, while preventing disorder of the healthy part, and continuing treatment to the lesioned part.

### Means for solving the problems

The photodynamic therapy apparatus is the photodynamic therapy apparatus for treating the lesioned part existing at a deep part of a living body, using a light-sensitive substance which is activated by a light having a peak intensity within a predetermined range, but almost not activated by a light having the peak intensity out of the predetermined range, having: an irradiation means which irradiates, onto the living body, pulsed light having wavelength which is capable of activating the light-sensitive substance; a detection means which detects activation of the light-sensitive substance; and a control means which controls the peak intensity of the light, based on result of detection obtained by the detection means so that the light-sensitive substance is not activated at the healthy part existing at a shallower part than the lesioned part, and the light which reaches the lesioned part has the peak intensity within the predetermined range.

### Advantage of the Invention

According to the photodynamic therapy apparatus of the present invention, activation of a light-sensitive substance is detected, and peak intensity of a light is controlled so that the light-sensitive substance is not activated at the healthy part existing at a shallower part than the lesioned part, and a light which reaches the lesioned part has the peak intensity within the predetermined range. Therefore, there is no damage to cells of the healthy part, by activation of a light-sensitive substance; namely the healthy part can be preserved.

### Best Embodiments to carry out the Invention

Firstly, mechanism of the photodynamic therapy (PDT) will be explained, and subsequently embodiments of the present invention for executing the PDT will be explained.

### PDT

The PDT is a method for treating a localized lesioned part like a cancer or the like.

In the PDT, firstly a light-sensitive substance (a PDT agent) is introduced into a living body by an intravenous injection or the like. The PDT agent is the medical agent having a property accumulating at the lesioned part, and activated by irradiation of a light having intensity within a predetermined range. The PDT agent includes a porphyrin derivative or a chlorine-based substance, and ATX-S10Na (II) or the like is included; the PDT agent is accumulated at the lesioned part in high concentration, due to accumulation property of the PDT agent. The accumulation requires certain time, therefore the medical agent may directly be injected to a part where the lesioned part is existing, or may directly be taken in by a cell.

After accumulation of the PDT agent at the lesioned part, a light is irradiated, which is capable of activating the PDT agent. The light, which is capable of activating the PDT agent, includes, for example, a light of semiconductor laser, dye laser, optical parametric oscillator or the like.

When the PDT agent is activated by pulsed light, oxygen around the PDT agent is activated. Oxygen thus activated has strong oxidative ability as singlet oxygen. By oxidative ability of singlet oxygen, cells or blood vessels of the lesioned part are damaged, and thus the living body is treated.

In the PDT, the PDT agent accumulates, and treatment is executed at a part irradiated with a light having peak intensity within a predetermined range. In this case, treatment cannot properly be fulfilled under condition of too high peak intensity and also too low peak intensity than a predetermined range. The range of peak intensity of a light to activate the PDT agent differs depending on the PDT agent to be introduced.

An example of the range of peak intensity of a light to activate the PDT agent will be shown.

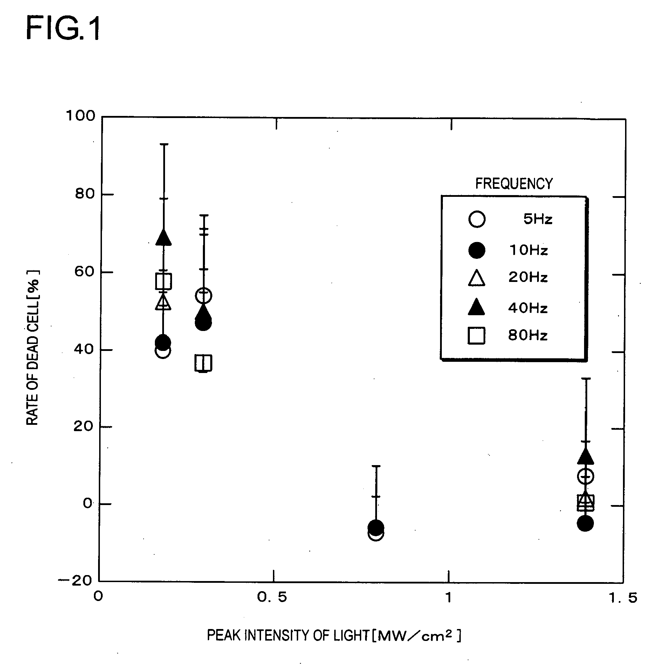

FIG. 1 is a drawing showing relation between peak strength of a light and a rate of dead cell. The rate of dead cell is rate of cells damaged by activation and action of the PDT agent, and increases with activation of the PDT agent.

Result shown in FIG. 1 was obtained under the following conditions. A XeCl excimer dye laser (wavelength: 669 ± 3 nm, full width at half maximum of pulse: 7 ns) was used. A light was vertically irradiated using a quartz fiber with a diameter of 600 µm. Irradiation was carried out by changing a light irradiation condition of a pulse peak intensity of from 0.17 to 1.4 MW/cm², and a repetition frequency of from 5 to 80 Hz.

By referring to FIG. 1, it was found that the rate of dead cell reached 60% under condition of a pulse peak intensity of 0.17 MW/cm², and a repetition frequency of 80 Hz. On the other hand, under condition of a high intensity pulse excitation of 1. 4 MW/cm², the rate of dead cell was dramatically decreased in any of the repetition frequency. The rate of dead cell, namely activation rate of the PDT agent, is found not to depend on the repetition frequency but depend on the pulse peak intensity, and too high pulse peak intensity does not provide PDT effect.

In this way, a proper range is existing in peak intensity of a light to activate the PDT agent, and too high intensity is found ineffective for activation.

The PDT agent has property to accumulate at the lesioned part, however, it is existing also at the healthy part around the lesioned part, in lower concentration than at the lesioned part. Therefore, irradiation of a light having peak intensity within a predetermined range, at the healthy part around the lesioned part, may result in damage of the healthy part. Consequently, in the case where the healthy part is existing at a shallow part, and the lesioned part at a deeper part thereof, pulsed light, which has peak intensity higher than within a predetermined range in advance, is irradiated so as to provide peak intensity of equal to or higher than a predetermined range at the healthy part, and peak intensity within a predetermined range at the lesioned part. By this way, damage of the healthy part can be prevented.

However, even when pulsed light having high peak intensity is irradiated, peak intensity could become within a predetermined range at the healthy part, by variation of a living body or concentration variation of the PDT agent or the like, and thus damage the healthy part.

Explanation will be given below on embodiments to surely prevent damage of such the healthy part.

The embodiments of the present invention will be explained by referring to drawings.

### (A first embodiment)

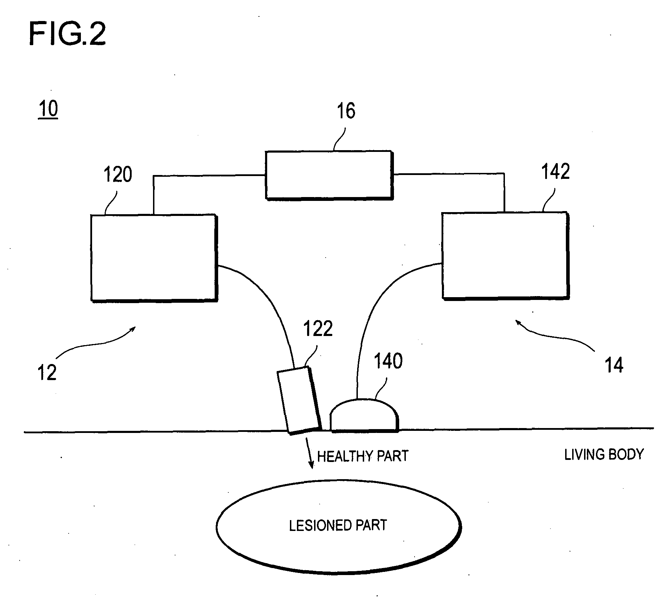

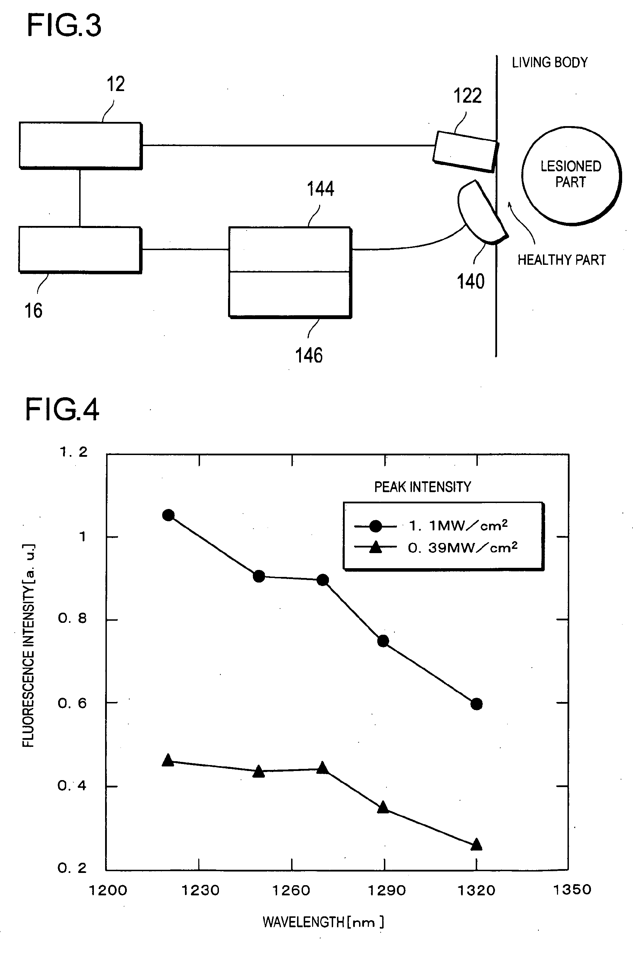

FIG. 2 is a drawing showing an outline structure of the photodynamic therapy apparatus in the present embodiment, and FIG. 3 is a drawing showing outline configuration of the detection device.

As shown in FIG. 2, the photodynamic therapy apparatus 10 has the irradiation device (irradiation means) 12, the detection device (detection means) 14, and the control device (control means) 16.

The irradiation device 12 has the irradiation main body 120 and the irradiation part 122. The irradiation main body 120 has a light source, and generates pulsed light with high peak intensity, of semiconductor laser, dye laser, or optical parametric oscillator. The irradiation part 122 is arranged at a surface of a living body near the lesioned part existing at a deep part of a living body. A pulsed light generated at the irradiation main body 120 is transmitted to the irradiation part 122, and irradiated toward an affected part from the irradiation part 122.

The detection device 14 has the light receiving part 140 and the detection main body 142. The light receiving part 140 is arranged with a light receiving surface at the surface of a living body toward the healthy part at a shallower part than the lesioned part. The light receiving part 140 receives a weak light called fluorescence emitted from singlet oxygen, which is generated in PDT. The detection main body 142 detects activation of the PDT agent, based on fluorescence received in the light receiving part 140 to transmit it to the control device 16.

The detection main body 142, as shown in FIG. 3, has the photoelectron multiplier 144 and the cooling device 146. The photoelectron multiplier 144 is an ultra-high sensitivity photo sensor, which is capable of amplifying and detecting weak fluorescence received. The cooling device 146 cools the photoelectron multiplier 144 to reduce noise caused by heat radiation emitted from surrounding devices like a photoelectron surface of the photoelectron multiplier 144 and the like.

The control device 16 is connected with the irradiation device 12 and the detection device 14. The control device 16 controls peak intensity of pulsed light irradiated by the irradiation device 12, based on detection of activation of the PDT agent by the detection device 14.

More detailed explanation will be given on measurement of fluorescence by the detection device 14, and detection of activation of the PDT agent.

FIG. 4 is a drawing measuring a spectrum including a fluorescence peak of singlet oxygen.

For example, a solution of the PDT agent with a concentration of 40 µg /ml is irradiated with a light under conditions of a pulse peak intensity of 0.39 MW/cm² and 1.1 MW/cm² and a repetition frequency of 80 Hz. Then a light with a wavelength range of from 1220 nm to 1320 nm is measured by the photoelectron multiplier 144, which is cooled by the cooling device 146. In this case, a peak around 1270 nm is observed in any of a pulse peak intensity of 0.39 MW/cm² and 1.1 MW/cm². This peak observed at this wavelength is known to be fluorescence emitted by the singlet oxygen, which is generated by activation of the PDT agent.

Therefore, in the case where the PDT is carried out under such conditions as above, by observation of fluorescence at a wavelength of near 1270 nm, and when a peak is observed, then determination is made that the PDT agent is activated.

According to this criterion, the detection device 14 is capable of detecting activation of the PDT agent.

Then, explanation will be given on operation of the photodynamic therapy apparatus 10.

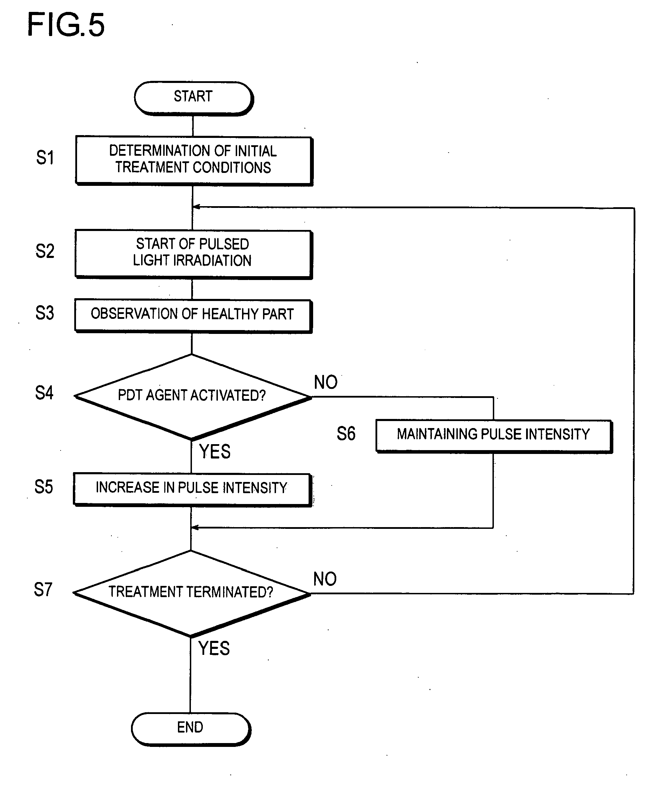

FIG. 5 is a flow chart showing an operation flow of the photodynamic therapy apparatus.

Firstly, initial conditions for treatment are input to the control device 16 (the step S1). The initial conditions for treatment include peak intensity, frequency, total energy density of pulsed light to be irradiated by the irradiation device 12, and concentration of the PDT agent supplied to a living body, and the like. These conditions are determined based on a state obtained by medical examination, in advance, of a living body, for example, location of the lesioned part and the like. In detail, depth of the lesioned part is measured, and initial peak intensity of pulsed light irradiated by the irradiation device 12 is determined so that peak intensity of pulsed light at the lesioned part is within a predetermined range for activation of the PDT agent.

Based on the initial conditions for treatment, after the irradiation part 122 of the irradiation device 12 is arranged at the vicinity of the lesioned part, the control device 16 makes the irradiation device 12 irradiate pulsed light toward the lesioned part under control of the irradiation device 12 (the step S2). By irradiation of pulsed light, the PDT agent is activated at the lesioned part, and cells at the lesioned part are damaged by the above-described mechanism.

During the PDT, the detection device 14 is arranged at the vicinity of the healthy part nearer to the surface of a living body than the lesioned part so as to observe a state of the healthy part and detect activation of the PDT agent (the step S3). The detection device 14 detects whether fluorescence of singlet oxygen activated by the PDT agent generates from the healthy part, to detect activation of the PDT agent.

The control device 16 determines whether or not the PDT agent is activated at the healthy part, based on fluorescence detected by the detection device 14 (the step S4). How the control device 16 determines whether or not the PDT agent is activated at the healthy part, based on fluorescence, will be explained later.

In the case where the PDT agent is activated (the step S4: YES), because the healthy part is damaged, the control device 16 makes the irradiation device 12 increase peak intensity of pulsed light by controlling the irradiation device 12 (the step S5); in this way, intensity of pulsed light permeating the healthy part is increased, by which activation of the PDT agent at the healthy part can be prevented. Namely, intensity of pulsed light, which reaches the lesioned part, is within a predetermined range, which is capable of activating the PDT agent. Explanation will be given later on to what degree the intensity of the pulsed light is increased.

In the case where the PDT agent is not activated (the step S4: NO), because the healthy part is not damaged, the control device 16 maintains present peak intensity of pulsed light (the step S6).

Then, the control device 16 determines whether or not treatment is over, based on initial treatment conditions including treatment time and the like (the step S7). In the case where the treatment is not over (the step S7: NO), processing from the step 2 is repeated. In the case where the treatment is over (the step S7: YES), the photodynamic therapy is terminated.

Next, explanation will be given on criterion on whether or not the PDT agent is activated, in the above step S4, along with to what degree the intensity of the pulsed light is increased, in the above step S5, in the case where the PDT agent is activated.

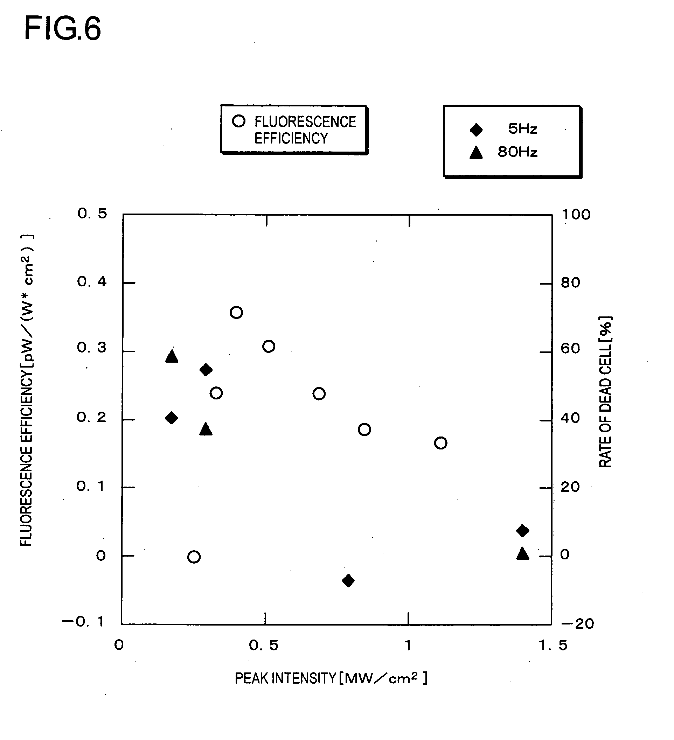

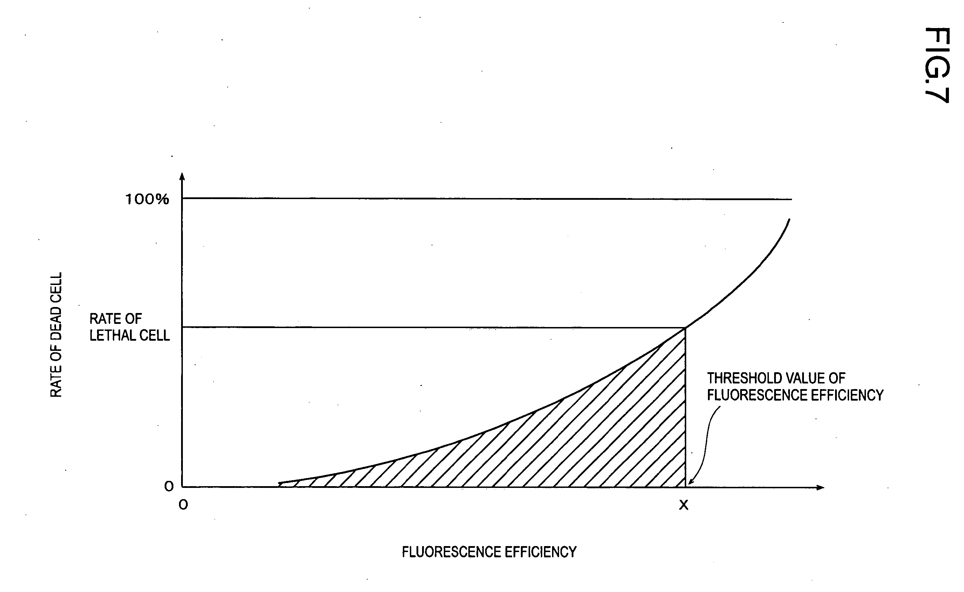

FIG. 6 is a drawing showing relations between fluorescence efficiency and peak strength, and relations between fluorescence efficiency and rate of dead cell, and FIG. 7 is a drawing showing relation between fluorescence efficiency and rate of dead cell.

In FIG. 6, the abscissa axis represents peak intensity of a light; the left side ordinate axis represents fluorescence efficiency; and the right side ordinate axis represents rate of dead cell. Here, fluorescence efficiency is defined as ratio of intensity of fluorescence from free radical, which is generated by activation of the PDT agent, to intensity of an incident light to a living body by the irradiation device 12. In FIG. 6, an open circle shows relation between the peak intensity and the fluorescence efficiency; and a filled lozenge mark (at 5 Hz) and a filled triangle mark (at 80 Hz) show relation between the peak intensity and the rate of dead cell.

In FIG. 7, the abscissa axis represents fluorescence efficiency; the ordinate axis represents rate of dead cell. Here, rate of lethal cell of the ordinate axis is defined as a ratio of rate of dead cell, which are not capable of recovering function as a tissue, in the case where a certain ratio or more of cells configuring a tissue died by action of the PDT agent.

By referring to an open circle in FIG. 6, in the case of excitation under peak intensity of equal to or higher than 0.4 MW/cm², higher peak intensity is found to more reduce the type II reaction of the PDT; namely fluorescence efficiency is reduced because generation of singlet oxygen is suppressed. Fluorescence efficiency here is defined as fluorescence power density of singlet oxygen detected, relative to power density of a light irradiated. By referring to the filled lozenge mark and the filled triangle mark, higher peak intensity is found to reduce rate of dead cell. In consideration of these tendencies, higher peak intensity is found to decrease both fluorescence efficiency and rate of dead cell.

This result shows presence of positive correlation between fluorescence efficiency and rate of dead cell, as shown in FIG. 7.

FIG. 7 shows a threshold value x of fluorescence efficiency for providing rate of lethal cell. Fluorescence efficiency equal to or smaller than the threshold value x provides cell recovery; therefore, the control device 16 does not determine that the PDT agent is activated at the healthy part, in the case where fluorescence efficiency detected by the detection device 14 is smaller than the threshold value x. On the contrary, in the case where the fluorescence efficiency is equal to or larger than the threshold value x, determination is made that the PDT agent is activated at the healthy part. In this way, whether or not the fluorescence efficiency is equal to or larger than the threshold value x is the criterion of whether or not the PDT agent is activated, in the step S4.

In the case where the PDT agent is activated, by attaining smaller value than the threshold value x, rate of lethal cell can be prevented. Therefore, the control device 16 controls the irradiation device 12 so that fluorescence efficiency is smaller than the threshold value x. Here, as shown in FIG. 6, larger peak intensity of pulsed light more reduces fluorescence efficiency. Therefore, the control device 16 controls the irradiation device 12 to increase peak intensity of pulsed light, till the fluorescence efficiency lower than the threshold value x is detected. This is the criterion to what degree intensity of pulsed light is increased.

In this way, the control device 16 is capable of automatically controlling peak intensity of pulsed light to be within a predetermined range, which is capable of activating the PDT agent in the lesioned part, by controlling the fluorescence efficiency to be smaller than the threshold value x.

Effects in the first embodiment are as follows.

Based on fluorescence of singlet oxygen, the type II reaction generated by activation of the PDT agent is detected. Then, in the case where activation of the PDT agent at the healthy part is detected, peak intensity of a light irradiated is increased. Therefore, peak intensity of a light in permeating the healthy part, existing at a shallower part than the lesioned part, is increased than a predetermined range for activation of the PDT agent. In this way, because a light-sensitive substance is not activated at the healthy part, cells at the healthy part are not seriously damaged; namely, the healthy part is preserved.

By detectingfluorescence generating from singlet oxygen, the type II reaction can directly be detected, which is caused by activation of a light-sensitive substance.

The photodynamic therapy apparatus 10, because both of the irradiation part 122 and the light receiving part 140 are arranged toward the surface side of a living body, can receive fluorescence generated inside the living body, without directly receiving a light irradiated. In the photodynamic therapy apparatus 10, the light receiving part 140 is arranged toward the surface side of a living body so that fluorescence generated inside the living body can be received and not a light irradiated.

It should be noted that, the detection device 14 may be provided with a spectroscopic part instead of, or in addition to the above configuration.

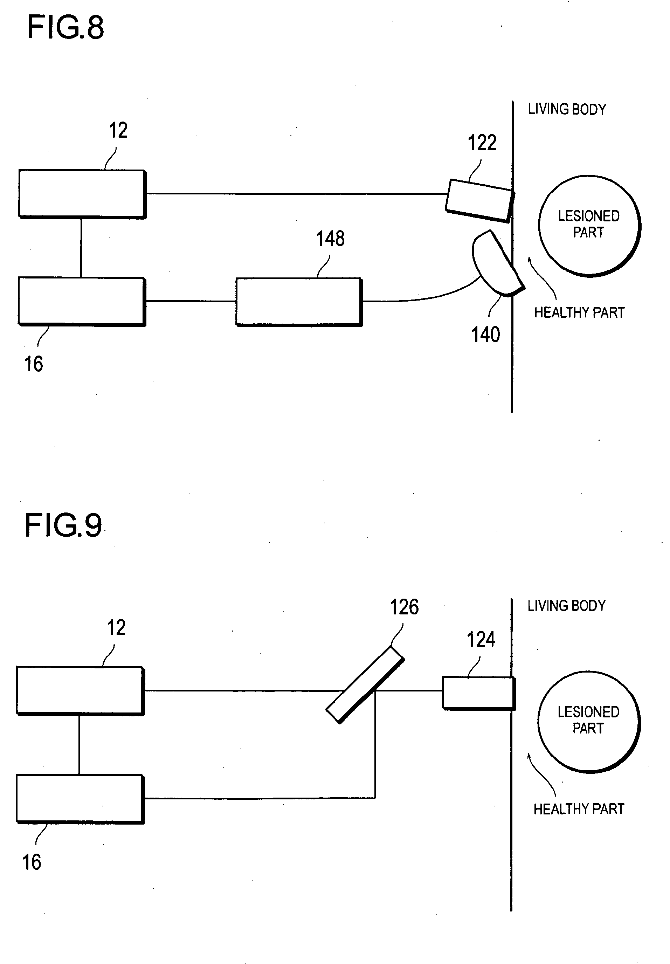

FIG. 8 is a drawing showing a detection device provided with a spectroscopic part. In the detection device 14, the light receiving part 140 is provided with the spectroscopic part 148. The spectroscopic part 148 is, for example, a diffraction lattice, a prism, a filter or a spectrometer systematized thereof or the like. By the spectroscopic part 148, only a light with a wavelength of around 1270 nm can be detected.

In addition, in the photodynamic therapy apparatus 10, the irradiation part 122 of the irradiation device 12, and the light receiving part 140 of the detection device 14 are separately configured, however, the configuration is not limited thereto; the irradiation part 122 and the light receiving part 140 may be configured as a one piece structure.

FIG. 9 is an outline block diagram of the photodynamic therapy apparatus configured with an irradiation part and a light receiving part as one piece.

As shown in FIG. 9, an irradiation part and a light receiving part are configured in a unified manner, as the tip part 124. In this case, a light guide for the irradiation device 12, and the detection device 14 is commonly used; therefore, the optical path control device 126 like a dichroic mirror or the like is arranged between the tip part 124, and the irradiation main body 120 and the control device 16. The optical path control device 126 transmits pulsed light (a wavelength of 670 nm) irradiated by the irradiation main body 120, but reflects fluorescence (a wavelength of 1270 nm) from a living body and introduces toward the control device 16.

In this way, by arrangement of the optical path controlling device 126, the tip part 124 common to the irradiation device 12 and the detection device 14 can be used, which contributes to simplification of an device. Furthermore, arrangement of an irradiation position and a light receiving position in close vicinity is capable of more surely detecting activation of the PDT agent at the healthy part.

It should be noted that, as for configuration of the photodynamic therapy apparatus, other configurations may also be considered.

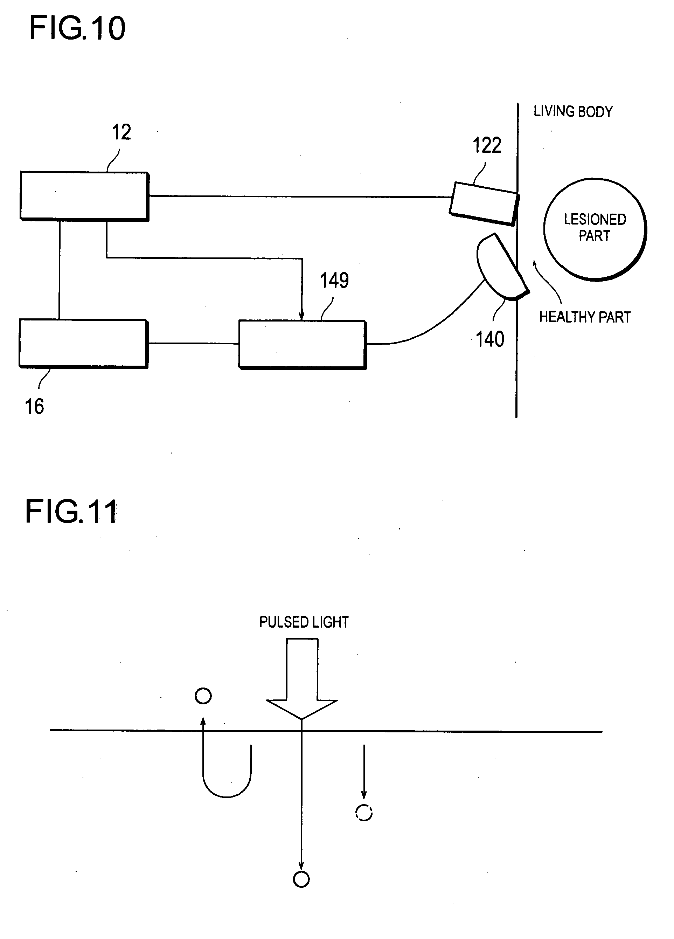

FIG. 10 is a drawing showing other configuration of the photodynamic therapy apparatus.

As shown in FIG. 10, repetition frequency of pulsed light from the irradiation device 12 may also be taken out as a reference signal and input to the demodulation part 149 installed at the detection device 14. In this case, the demodulation part 149 executes lock-in detection from a light received by the light receiving part 140, based on the reference signal. In this way, demodulation in light receiving is capable of reducing noise and surely detecting activation of the PDT agent.

### (A second embodiment)

In the first embodiment, activation of the PDT agent is detected based on fluorescence generating from singlet oxygen. However, in the second embodiment, activation of the PDT agent is detected, by detecting a light once irradiated to a living body and scattered inside the living body and returned, instead of fluorescence.

Explanation will be given on detection mechanism of activation of the PDT agent in the second embodiment.

FIG. 11 is a drawing showing detection mechanism of activation of the PDT agent.

As shown in FIG. 11, pulsed light is irradiated toward a living body. The irradiated light travels inside the living body as shown by an arrow mark. Like pulsed light shown by an arrow mark at the right side of the drawing, a part of the pulsed light is absorbed by the PDT agent. In addition, like pulsed light shown by an arrow mark at the center of the drawing, a part of the pulsed light transmits the living body; and like pulsed light shown by an arrow mark at the left side of the drawing, a part of the pulsed light is repeatedly scattered inside the living body and emitted again outside a living body from the surface of a living body. Hereafter, such a light emitted again outside a living body from inside a living body is referred to as a backscattering light.

A light scattered at a shallow part of a living body is known to become, and observed as, a backscattering light at a position near an incident position of pulsed light irradiated; on the other hand, a light scattered at a deep part of a living body is known to become, and observed as, a backscattering light at a position apart from an incident position. Therefore, in the case where the lesioned part is existing at a deep part, and the healthy part is existing at a shallow part thereof, observation of a backscattering light at a position near an incident position is capable of detecting a backscattering light of pulsed light scattered at the healthy part.

Amount of a backscattering light varies depending on a living body itself, and concentration and distribution of the PDT agent contained in the living body. Activation of the PDT agent at a shallow part of a living body damages the PDT agent existing at a shallow part of a living body by singlet oxygen generated, resulting in losing activation of the PDT agent damaged, and reducing concentration of the PDT agent. This phenomenon is called "bleaching". Occurrence of bleaching in the healthy part at a shallow part of a living body reduces pulsed light to be absorbed by the PDT agent, resulting in increase in amount of a backscattering light.

In this way, activation of the PDT agent at a shallow part of a living body results in increase in amount of a backscattering light; by utilization of this result, in the second embodiment, activation of the PDT agent is detected, based on increase in amount of a backscattering light.

Configuration of the photodynamic therapy apparatus used in the second embodiment is similar to that shown in FIG. 2, however, action is different.

The irradiation device 12 irradiates pulsed light toward a living body so that peak intensity for activation of the PDT agent is obtained at the lesioned part. A part of the pulsed light is scattered inside the living body and emitted outside the living body as a backscattering light. The detection device 14 receives a backscattering light by the light receiving part 140. When amount of a backscattering light received increase, namely, activation of the PDT agent is detected at a shallow part of a living body, the control device 16 powers up pulsed light, to be output from the irradiation main body 120, so as to prevent activation of the PDT agent at a shallow part of the living body.

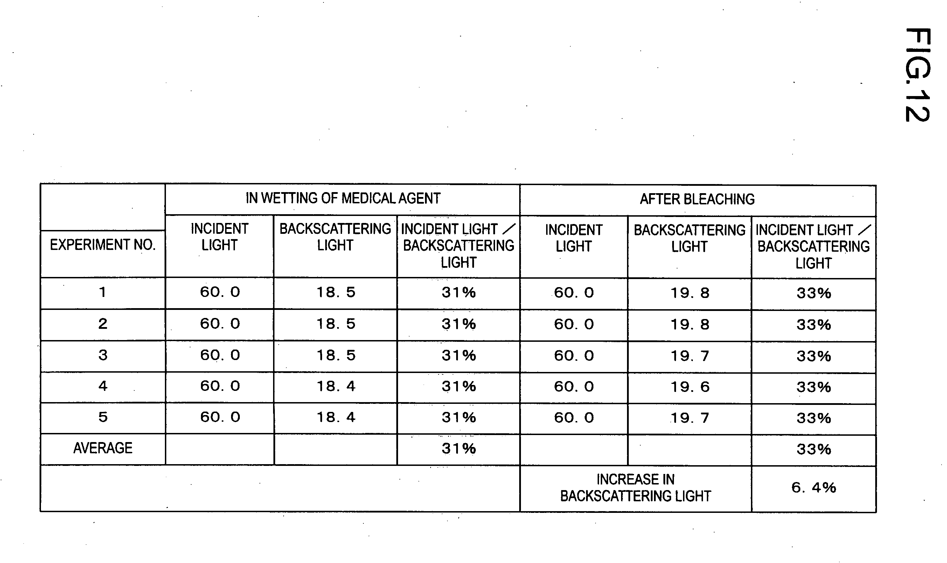

FIG. 12 is a drawing showing increase in backscattering light, in the case where the PDT agent is activated at a shallow part of a living body.

A pulsed light was irradiated to cells wetted with the PDT agent, and ratio of a backscattering light at this time was detected. Subsequently, pulsed light was irradiated to cells showing bleaching by generation of activation of the PDT agent, to detect ratio of a backscattering light. Similar experiments were repeated 5 times.

Ratio of amount of a backscattering light relative to amount of an incident pulsed light to a living body, before generation of bleaching, was 31% in average; on the other hand, ratio of amount of a backscattering light relative to amount of an incident pulsed light to a living body, after generation of bleaching, was 33% in average. Comparison of these values shows increase by 2%, namely increase in a backscattering light by (2/31) x 100 = 6.4%.

As described above, activation of the PDT agent is found to increase a backscattering light; namely, detection of increase in a backscattering light is found to be capable of detecting activation of the PDT agent.

Increase in a backscattering light is caused, as described above, by lowering of concentration of the PDT agent. Detection of a backscattering light is also capable of detecting concentration of the PDT agent. Therefore, activation of the PDT agent can also be detected, based on concentration of the PDT agent.

### (A third embodiment)

In the first embodiment, activation of the PDT agent is detected based on fluorescence generating from singlet oxygen. However, in the third embodiment, activation of the PDT agent is detected, by detecting a light permeating a living body, instead of fluorescence.

Explanation will be given as follows on detection mechanismof activation of the PDT agent in the third embodiment. Activation of the PDT agent generates bleaching and decreases concentration of the PDT agent, resulting in increase in amount of pulsed light passing through a living body without being absorbed by the PDT agent. In the third embodiment, pulsed light passing through a living body is detected by a detection device, which is punctured into a living body. In this way, ratio of absorption (hereafter referred to as absorbance) of pulsed light by the PDT agent is measured to detect change in concentration of the PDT agent. Activation of the PDT agent can be detected, based on decrease in concentration of the PDT agent.

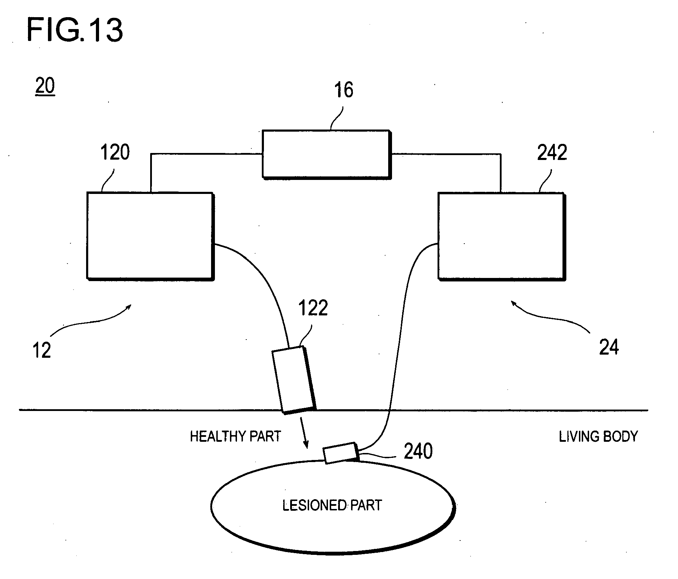

FIG. 13 is an outline block diagram of the photodynamic therapy apparatus relevant to the third embodiment. As for configuration similar to that in the first embodiment, similar reference number is attached and explanation thereof will be omitted.

The photodynamic therapy apparatus 20 has the irradiation device 12, the control device 16 and the detection device 24.

The detection device 24 has the puncture part 240 and the detection main body 242.

The puncture part 240 is formed needle-likely or plate-likely, and is punctured into a little shallower part than the lesioned part, which is irradiated with pulsed light by the irradiation part 122. The puncture part 240 is formed with a light receiving surface to fulfill a role of the light receiving part to receive pulsed light from the irradiation part 122.

The puncture part 240 is connected to the detection main body 242. The detection main body 242 measures absorbance of pulsed light, based on pulsed light received in the puncture part 240. The detection main body 242 calculates ratio of present absorbance relative to absorbance just after irradiation start, as absorption decrease.

Then, based on the absorption decrease, decrease in concentration of the PDT agent and also activation of the PDT agent are detected. The detection main body 242 transmits the detection result to the control device 16.

The control device 16, upon detection of activation of the PDT agent, powers up pulsed light to be output from the irradiation main body 120, thus preventing activation of the PDT agent at a shallow part of the living body.

As described above, detection of decrease in concentration of the PDT agent is capable of surely detecting activation of the PDT agent in the healthy part.

It should be noted that, the puncture part 240 may not be punctured on treatment; the puncture part 240 may surgically be implanted inside a living body in advance of treatment instead of being punctured.

In addition, in the above embodiment, activation of the PDT agent is detected by detection of the pulsed light for treatment, by the detection device 24, however, a light having wavelength to be absorbed by the PDT agent may separately be permeated a living body so as to be received by the puncture part 240 arranged inside a living body. In this case, the irradiation device 12 is provided with an inspection light irradiation part, which irradiates an inspection light.

For example, a light having a wavelength of from 122 to 670 nm is irradiated for treatment, and a light having a wavelength of 405 nm is irradiated for inspection from the separate inspection light irradiation part.

### (A fourth embodiment)

In the first embodiment, activation of the PDT agent is detected based on fluorescence generating from singlet oxygen. However, in the fourth embodiment, activation of the PDT agent is detected, by detecting oxygen partial pressure of the living body, instead of fluorescence.

Explanation will be given as follows on detection mechanism of activation of the PDT agent in the fourth embodiment. Activation of the PDT agent generates singlet oxygen. Generation of singlet oxygen consumes oxygen and thus decreases oxygen concentration, namely decreases oxygen partial pressure. Therefore, detection of decrease in oxygen partial pressure is capable of detecting activation of the PDT agent. As a method for measuring oxygen partial pressure, a measurement method for a percutaneous oxygen partial pressure by a Clark-type oxygen electrode is known.

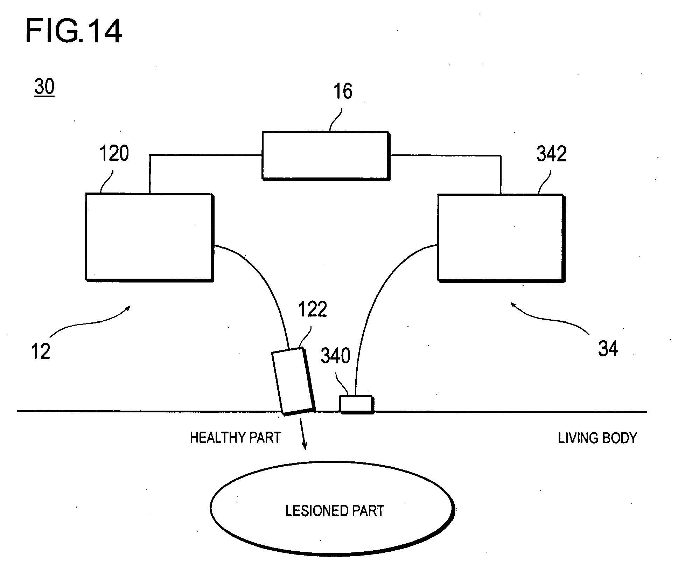

FIG. 14 is an outline block diagram of the photodynamic therapy apparatus relevant to the fourth embodiment. As for configuration similar to that in the first embodiment, similar reference number is attached and explanation thereof will be omitted.

The photodynamic therapy apparatus 30 has the irradiation device 12, the control device 16 and the detection device 34.

The detection device 34 has the oxygen electrode 340 and the detection main body 342.

The oxygen electrode 340 is arranged so as to contact a living body at the vicinity of the irradiation part 122. The oxygen electrode 340 measures oxygen partial pressure. The detection main body 342 detects activation of the PDT agent at a shallow part of the living body by detecting decrease in oxygen partial pressure. The detection main body 342 transmits the detection result to the control device 16.

The control device 16, upon detection of activation of the PDT agent, powers up pulsed light to be output from the irradiation main body 120, thus preventing activation of the PDT agent at a shallow part of the living body.

As described above, detection of decrease in oxygen partial pressure is capable of surely detecting the activation of the PDT agent at the healthy part.

### (A fifth embodiment)

In the first embodiment, activation of the PDT agent is detected based on fluorescence generating from singlet oxygen. However, in the fifth embodiment, activation of the PDT agent is detected, by specifying oxygen concentration in the healthy part, by detecting phosphorescence emitted from the PDT agent, instead of fluorescence.

Explanation will be given as follows on detection mechanismof activation of the PDT agent in the fifth embodiment. Activation of the PDT agent generates singlet oxygen by energies of the PDT agent. Generation of singlet oxygen consumes oxygen and decreases local oxygen concentration around the PDT agent. Therefore, detection of decrease in oxygen concentration at the healthy part around the lesioned part is capable of detecting activation of the PDT agent at the healthy part. In this case, a range of measurement of decrease in local oxygen concentration is defined as a range having possibility for the medical agent to react with surrounding oxygen in irradiation of pulsed light. By assuming triplet lifetime (time during being activated) of the medical agent to be 1 ms, a range where local decrease in oxygen concentration can be measured is a local range of about 2.6 µm around the medical agent as a center.

Oxygen concentration here can be detected based on phosphorescence generating from the PDT agent. Phosphorescence is a light generated by the PDT agent itself, in the case where energies of activated PDT agent cannot be transferred to oxygen. Decrease in oxygen concentration around the PDT agent inhibits energy-transfer and thus increases probability of the PDT agent generating phosphorescence, and also improves intensity of phosphorescence as a whole.

Therefore, detection of increase in intensity of phosphorescence, which is generated at the healthy part, is capable of detecting decrease in oxygen concentration, by which activation of the PDT agent at the healthy part can be detected.

Explanation will be given on the photodynamic therapy apparatus of the fifth embodiment, where phosphorescence is detected.

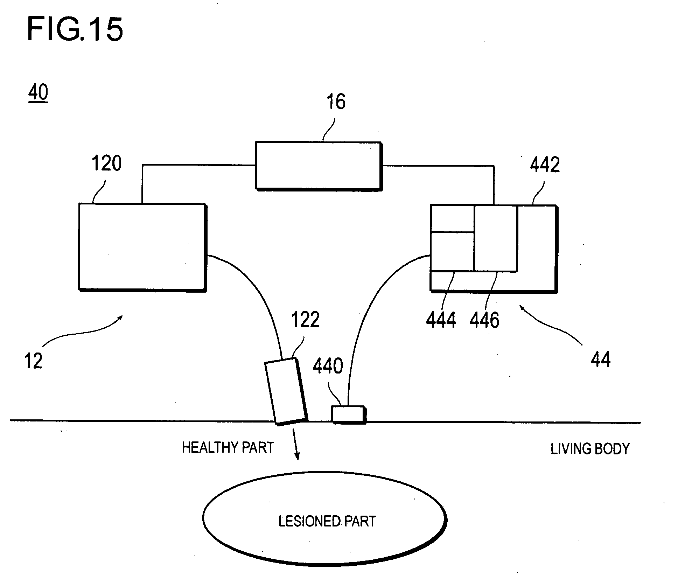

FIG. 15 is an outline block diagram of the photodynamic therapy apparatus relevant to the fifth embodiment. The photodynamic therapy apparatus relevant to the fifth embodiment has same configuration as in the first embodiment shown in FIG. 2 except configuration of a detection device. As for same configuration as that in the first embodiment, same reference number is attached and explanation thereof will be omitted.

In addition, FIG. 16 is a drawing showing generation timing of phosphorescence; FIG. 17 is a drawing showing waveform data of phosphorescence generated by an experiment; FIG. 18 is a drawing explaining definition of lifetime of phosphorescence generated; FIG. 19 is a drawing showing correlation between lifetime of phosphorescence and oxygen concentration; and FIG. 20 is a drawing showing waveforms of phosphorescence generating in different oxygen concentrations.

The photodynamic therapy apparatus 40 has the irradiation device 12, the control device 16 and the detection device 44.

The detection device 44 has the light receiving part 440 and the detection main body 442.

The light receiving part 440 is arranged so as to contact a living body at the vicinity of the irradiation part 122. The light receiving part 440 receives a light generated at the healthy part. It should be noted that phosphorescence may generate also at the lesioned part, however, because phosphorescence is extremely weak, phosphorescence generated at the lesioned part cannot be detected by a light receiving part 440. The detection main body 442 detects phosphorescence from a light received by the light receiving part 440. Therefore, the detection main body 442 further has the spectroscopic part 444 and the photoelectron multiplier 446 provided with a gate.

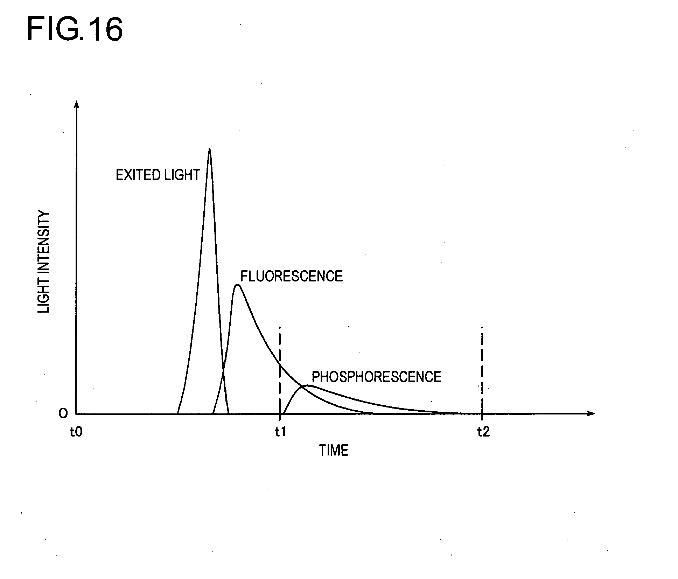

The spectroscopic part 444 is, for example, a diffraction lattice, a prism, a filter or a spectrometer systematized thereof, or the like. By the spectroscopic part 444, only a light with wavelength around that of phosphorescence can selectively be detected. Peak wavelength of phosphorescence has value between wavelength of fluorescence emitted from the PDT agent (see FIG. 16), and wavelength (for example, 762 nm) of a light emitted when singlet oxygen moves back toward ground state oxygen.

The photoelectron multiplier 446 provided with a gate is an ultra-sensitive optical sensor, which is capable of amplifying and detecting extremely weak phosphorescence taken-in, under limiting time for taking-in a light from the spectroscopic part 44, by open-close operation of the gate (a light receiving time control part).

Reason for limiting time for taking-in a light is as follows. As shown in FIG. 16, phosphorescence is a light generated subsequent to an excited light and fluorescence. An excited light is the pulsed light irradiated from the irradiation part 122. Fluorescence shown in FIG. 16 generates from the PDT agent introduced into a living body, different from fluorescence generating from singlet oxygen, which is detected in the first embodiment. Phosphorescence is a light generating in direct emission as energies from the PDT agent, in the case where energies cannot be transferred from the PDT agent to oxygen, because of absence of oxygen molecules around.

In this way, phosphorescence generates in timing different from an excited light and fluorescence, and is much weaker than an excited light and fluorescence, to greater extent than shown in the drawing. Therefore, the photoelectron multiplier 446 provided with a gate opens the gate in timing matching with generation of fluorescence to easily specify generation of fluorescence.

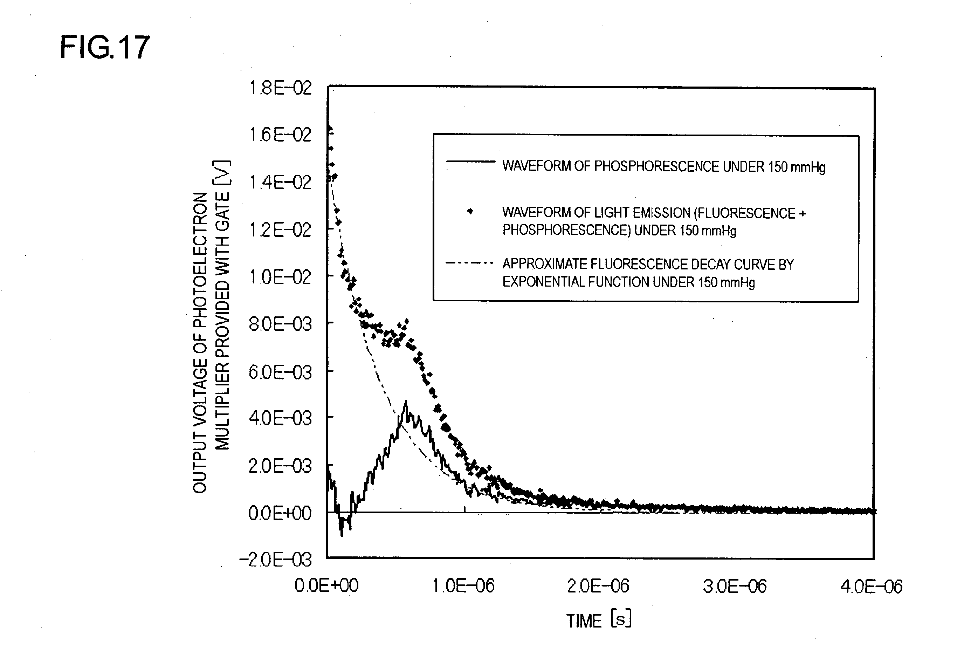

Phosphorescence, as shown in FIG. 16, mainly generates between time t1 and t2 measured from time t0, when a trigger signal of pulsed light generation is input to the irradiation device 12. Therefore, the photoelectron multiplier 446 provided with a gate opens the gate only from the time t1 to t2 to detect phosphorescence. Timing of gate opening differs depending on a measurement device. For example, as shown in FIG. 17, in the case where measurement result of waveform of light emission (integration number of 1000 times) was obtained in irradiation of pulsed light with a wavelength of 710 nm, timing till gate opening, namely, time from t0 to t1, was from 217 . 993 to 218.598 µs. In addition, time during gate opening, namely time from t1 to t2, was from 24.007 to 24.607 µs. As is understood by FIG. 17, because lifetime of phosphorescence is about several, this time during gate opening is enough for measurement. The detection device 44 transmits the detection result of a light to the control device 16.

The control device 16 receives the detection result of a light detected by the detection device 44, and acts as follows.

(1) Firstly, the control device 16 calculates lifetime of phosphorescence detected in the detection device 44. (2) The control device 16 specifies oxygen concentration in the healthy part from lifetime of phosphorescence thus calculated. (3) The control device 16 detects whether or not activation of the PDT agent is generated at the healthy part, based on oxygen concentration thus specified. The control device 16, upon detection of activation of the PDT agent, powers up pulsed light to be output from the irradiation main body 120, thus preventing activation of the PDT agent at a shallow part of the living body.

Explanation will be given on detailed operation procedure of the control device 16 in sequence.

(1) Procedure to calculate lifetime of phosphorescence is as follows.

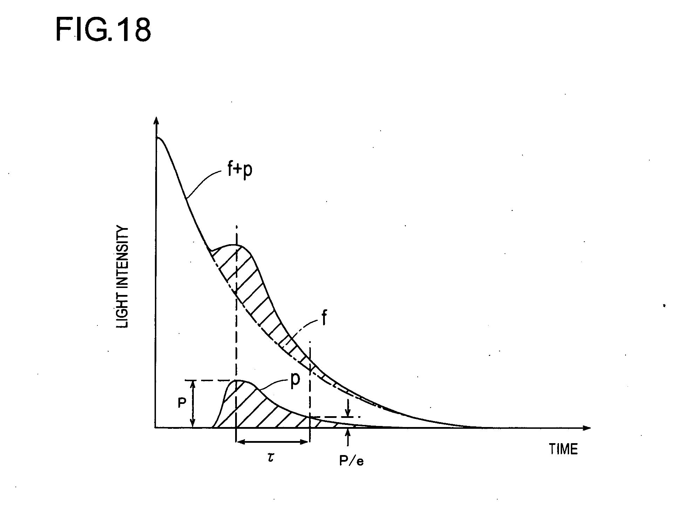

The control device 16, by analysis of a signal as it is, which is transmitted from the detection device 44, obtains a waveform f + p, shown in the upper side of FIG. 18. This waveform f + p is a graph measured in a mixed state of fluorescence and phosphorescence of the PDT agent. In the waveform f + p, a part with a light intensity a little improved at the center corresponds to phosphorescence.

The control device 16 complements a part for generation of phosphorescence, by using a part excluding a part for phosphorescence from the waveform f + p, namely using values before and after phosphorescence appears, to prepare a fitting curve f shown by a dashed line. The fitting curve f is a graph showing only intensity of fluorescence. Then, the control device 16 calculates difference between the graph f + p, and the fitting curve f to prepare graph p showing only phosphorescence.

The control device 16 determines a peak P of the graph p thus prepared, to calculate time τ for light intensity to reduce from the peak P down to value obtained by dividing the peak P with e; the control device 16 defines this time τ as lifetime of phosphorescence.

(2) Procedure to specify oxygen concentration in the healthy part from lifetime of phosphorescence is as follows.

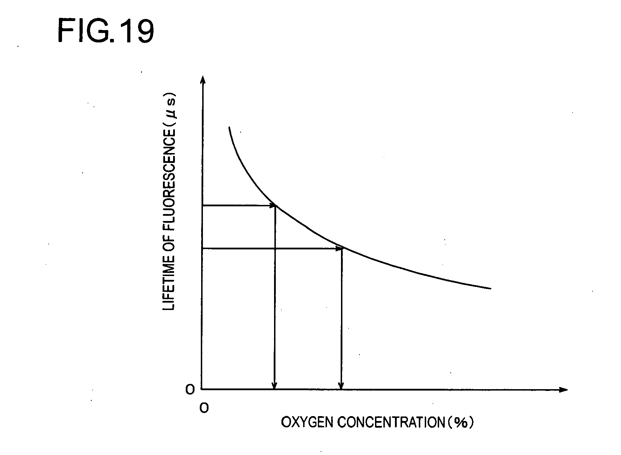

In the control device 16, as shown in FIG. 19, correlation, between lifetime of phosphorescence and oxygen concentration has been memorized in advance. This correlation can experimentally be determined.

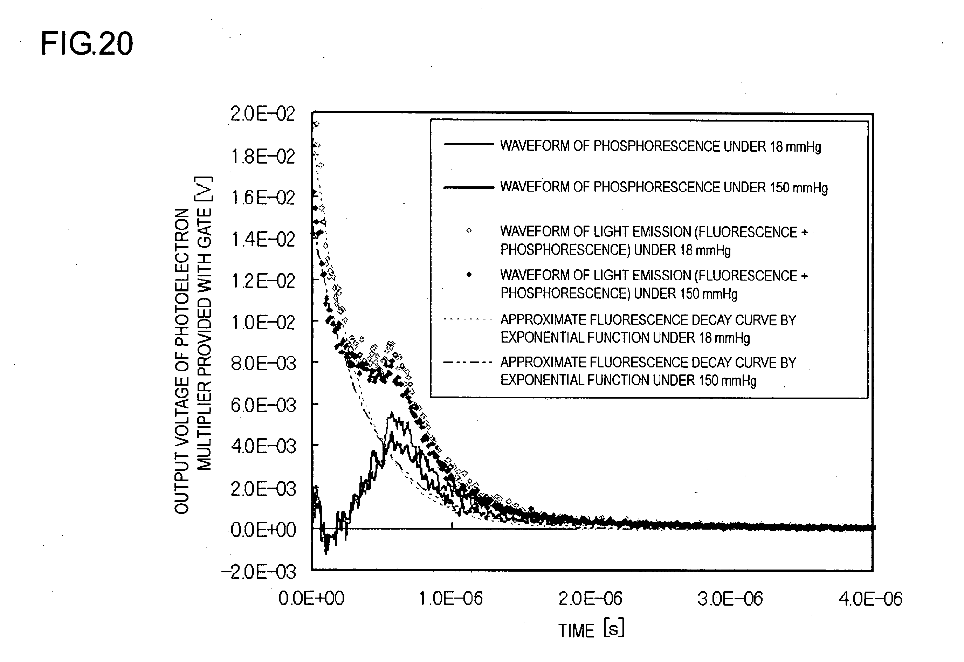

For example, as shown in FIG. 20, waveforms of phosphorescence are different between the case where oxygen concentration is 18 mmHg and the case where oxygen concentration is 150 mmHg. It should be noted that, measurement result shown in FIG. 20 is for waveform of light emission (integration number of 1000 times) obtained by irradiation of pulsed light with a wavelength of 710 nm, to a living body. In accordance with the above calculation procedure of lifetime of phosphorescence, each of the fitting curves is determined, to prepare a graph showing intensity of only phosphorescence and to calculate lifetime. It was found that lifetime of phosphorescence was 0.36 µs in the case where oxygen concentration was 18 mmHg; and lifetime of phosphorescence was 0.34 µs in the case where oxygen concentration was 150 mmHg. Lifetime of phosphorescence differed as large as 0.02 µs by difference in oxygen concentration. In this way, correlation shown in FIG. 19 is determined by repeating experiments.

The control device 16 specifies oxygen concentration by fitting lifetime of phosphorescence thus calculated to the correlation shown in FIG. 19.

(3) Procedure to detect activation of the PDT agent at the healthy part, based on oxygen concentration specified is as follows.

In the control device 16, oxygen concentration value as criterion on whether or not the PDT agent is activated, has been input in advance. Therefore, the control device 16 determines whether or not the oxygen concentration thus specified is equal to or higher than the criterion, or below. In the case where the oxygen concentration is below the criterion, it is determined that oxygen is consumed also in the healthy part, and activation of the PDT agent is generated there.

As described above, in the fifth embodiment, by detection of phosphorescence generating at the healthy part, oxygen concentration is specified, which is capable of detecting activation of the PDT agent at the healthy part. In particular, because lifetime of phosphorescence becomes longer inversely proportion to decrease in oxygen concentration at the healthy part around the lesioned part, decrease in local oxygen concentration around the lesioned part can be specified; therefore, activation of the PDT agent at the healthy part can be detected in better precision.

In addition, in the detection device 44, filtering is executed at wavelength including phosphorescence, by the spectroscopic part 444, and furthermore a light taking-in time is limited by the gate of the photoelectron multiplier 446 provided with the gate; therefore, an excited light or fluorescence included in a light detected in the detection device 44 can be minimized, and thus detection precision of phosphorescence can be improved.

It should be noted that in the fifth embodiment, by determination of lifetime of phosphorescence, oxygen concentration is specified, however, the method is not limited thereto; oxygen concentration may be specified, based on relative light intensity of a phosphorescence pulse or a phosphorescence spectrum. In this case, correlation between intensity of the phosphorescence pulse or the phosphorescence spectrum, and oxygen concentration is experimentally determined in advance.

In addition, in the fifth embodiment, explanation was given on the photoelectron multiplier provided with a gate, as an example of a method for measuring phosphorescence, however, the method is not limited thereto; an optical shutter like a Pockels cell may be used to limit incident time for an exited light or fluorescence other than phosphorescence to enter the photoelectron multiplier.

As explained above in the first embodiment though the fifth embodiment, activation of the PDT agent at the healthy part is detected. Then, in the case where the PDT agent is activated at the healthy part, by increasing peak intensity of pulsed light to be irradiated, activation of the PDT agent is limited only at the lesioned part deeper than the healthy part. Therefore, only the lesioned part can be treated while surely preserving the healthy part at a shallower part.

Such a PDT can be applied to treatment of prostatic cancer existing at deep prostate while transurethrally preserving urethral tissue. In addition, it can be applied to treatment of capsula fibrosa preserved arteriosclerotic atheroma, to avoid risk of acute myocardial infarction.

It should be noted that, the first embodiment through the fifth embodiment are applied to a method for the photodynamic therapy, where the lesioned part at a deep part of a living body is treated by irradiation of a light with intentionally high peak intensity at the healthy part existing at a shallow part of a living body, so as not to activate the PDT agent at a shallow part of a living body, however, the present invention can be applied to other methods for the photodynamic therapy.

As other methods for the photodynamic therapy, for example, there is a method for treatment of the lesioned part by focusing a light having wavelength with 2 times of wavelength for activation of the PDT agent at the deep lesioned part of the living body to generate simultaneous two-photon excitation. In such a method for the photodynamic therapy, light intensity is increased at deep of the living body by focusing, while preserving light intensity low at the healthy part at shallow part of a living body to preserve the healthy part at a shallow part.

Therefore, in application of the present invention and in the case where activation of the PDT agent at a shallow part of a living body is monitored and activation is detected, intensity of pulsed light is weakened so that fluorescence efficiency by free radical is below a threshold value. In this way, activation of the PDT agent at a shallow part of a living body can be prevented, and treatment can be maintained under control of no damage reception at the healthy part.

### Brief Description of Drawings

FIG. 1 is a drawing showing relation between peak intensity of a light and rate of dead cell.
FIG. 2 is a drawing showing an outline structure of the photodynamic therapy apparatus in the present embodiment.
FIG. 3 is a drawing showing outline configuration of the present detection device.
FIG. 4 is a drawing showing a spectrum including a fluorescence peak of singlet oxygen.
FIG. 5 is a flow chart showing an operation flow of the photodynamic therapy apparatus.
FIG. 6 is a drawing showing relations between fluorescence efficiency and peak strength, and between fluorescence efficiency and rate of dead cell.
FIG. 7 is a drawing showing relation between fluorescence efficiency and rate of dead cell.
FIG. 8 is a drawing showing a detection device provided with a spectroscopic part.
FIG. 9 is an outline block diagram of the photodynamic therapy apparatus configured with an irradiation part and a light receiving part as one piece.
FIG. 10 is a drawing showing other configuration of the photodynamic therapy apparatus.
FIG. 11 is a drawing showing detection mechanism of activation of the PDT agent.
FIG. 12 is a drawing showing increase in a backscattering light, in the case where the PDT agent is activated at a shallow part of a living body.
FIG. 13 is an outline block diagram of the photodynamic therapy apparatus relevant to a third embodiment.
FIG. 14 is an outline block diagram of the photodynamic therapy apparatus relevant to a fourth embodiment.
FIG. 15 is an outline block diagram of the photodynamic therapy apparatus relevant to a fifth embodiment.
FIG. 16 is a drawing showing generation timing of phosphorescence.
FIG. 17 is a drawing showing waveform data of phosphorescence generated by an experiment.
FIG. 18 is a drawing explaining definition of lifetime of phosphorescence generated.
FIG. 19 is a drawing showing correlation between lifetime of phosphorescence and oxygen concentration.
FIG. 20 is a drawing showing waveforms of phosphorescence generating in different oxygen concentrations.

### Explanation of Reference No.

- 10, 20, 30, 40: photodynamic therapy apparatus
- 12: irradiation device
- 14, 24, 34, 44: detection device
- 16: control device
- 120: irradiation main body
- 122: irradiation part
- 124: tip part
- 126: light pass control device
- 128: modulation part
- 140; 440: light receiving part
- 142, 242, 342, 442: detection main body
- 144: photoelectron multiplier
- 146: cooling device
- 148, 444: spectroscopic part
- 149: demodulation part
- 240: puncture part
- 340: oxygen electrode
- 446: photoelectron multiplier provided with a gate

## Claims

1. The photodynamic therapy apparatus for treating the lesioned part existing at a deep part of a living body, using a light-sensitive substance which is activated by a light having a peak intensity within a predetermined range, but substantively not activated by a light having the peak intensity out of the predetermined range, comprising:
an irradiation means which irradiates, onto the living body, pulsed light having wavelength which is capable of: activating the light-sensitive substance;
a detection means which detects activation of the light-sensitive substance; and
a control means which controls the peak intensity of the light, based on result of detection obtained by the detection means so that the light-sensitive substance is not activated at the healthy part existing at a shallower part than the lesionedpart, and the light which reaches the lesioned part has the peak intensity within the predetermined range.

2. The photodynamic therapy apparatus according to claim 1, wherein the control means increases the peak intensity of the light, which is irradiated by the irradiation means, in the case where activation of the light-sensitive substance in the healthy part is detected by the detection means.

3. The photodynamic therapy apparatus according to claim 1 or 2, wherein the detection means detects activation of the light-sensitive substance by detecting fluorescence generated from singlet oxygen, which generates when the light-sensitive substance is activated.

4. The photodynamic therapy apparatus according to claim 3, wherein the irradiation means comprises an irradiation part which is arranged toward a surface side of a living body at the vicinity of the lesioned part, and irradiates the light, and the detection means receives the fluorescence from inside of the living body by a light receiving part which is arranged at the surface of the living body at the vicinity of the irradiation part, and set toward the surface side of the living body.

5. The photodynamic therapy apparatus according to claim 4, wherein the detection means further comprises a cooling part which cools the living body and the light receiving part.

6. The photodynamic therapy apparatus according to claim 1 or 2, wherein the irradiation means comprises an irradiation part which is arranged toward the surface side of the living body at the vicinity of the lesioned part, and irradiates the light, and the detectionmeans receives the light scattered inside the living body and emitted outside the living body, by a light receiving part which is arranged at the surface of the living body at the vicinity of the irradiation part, and set toward the surface side of the living body, and detects activation of the light-sensitive substance, based on increase in a light amount received.

7. The photodynamic therapy apparatus according to claim 4 or 6, wherein the detecting unit further comprises a demodulation part, which demodulates a light received.

8. The photodynamic therapy apparatus according to claim 1 or 2, wherein the detection means measures a concentration of the light-sensitive substance at the healthy part, and detects activation of the light-sensitive substance, based on decrease in the concentration.

9. The photodynamic therapy apparatus according to claim 1 or 2, wherein the detection means measures an oxygen partial pressure in the living body, and detects activation of the light-sensitive substance, based on decrease in the oxygen partial pressure.

10. The photodynamic therapy apparatus according to claim 1 or 2, wherein the detection means comprising:
a puncture part which punctures the living body at the vicinity of the irradiation part; and
a light receiving part which is formed on the puncture part, and receives an inspection light permeated the living body from the surface of the living body;
and detects activation of the light-sensitive substance, based on increase in amount of the inspection light received by the light receiving part.

11. The photodynamic therapy apparatus according to claim 10, wherein the inspection light is a light having wavelength which is capable of activating the light-sensitive substance, and irradiated by the irradiation means or irradiated by a separate inspection light irradiation means from the irradiation means.

12. The photodynamic therapy apparatus according to claim 1 or 2, wherein the detection means specifies an oxygen concentration in the healthy part, by detecting phosphorescence emitted from the light-sensitive substance in the healthy part, and detects activation of the light-sensitive substance, based on decrease in the oxygen concentration specified.

13. The photodynamic therapy apparatus according to claim 12, wherein the detection means further comprising:
a spectroscopic part which provides a spectrum of phosphorescence emitted from the light-sensitive substance; and
a light receiving time regulating part which receives the phosphorescence.
